# FASCICULE DE BREVET EUROPEEN

(11) **EP 0 758 546 B1**
(45) Date de publication et mention de la délivrance du brevet: **29.10.2003**
(21) Numéro de dépôt: 96401643.0
(22) Date de dépôt: 23.07.1996
(51) Int. Cl.: A61K 7/06

(54) **Composition cosmétique comprenant une dispersion aqueuse de polymère non ionique, utilisation et procédé de fabrication**
Kosmetische Zusammensetzung die eine wässrige nicht-ionische Polymerdispersion enthält, ihre Verwendung und Herstellungsverfahren
Cosmetic composition containing an aqueous dispersion of a nonionic polymer, utilisation and preparation

(30) Priorité: 11.08.1995 FR 9509772
(43) Date de publication de la demande: 19.02.1997
(73) Titulaire: L'OREAL, 75008 Paris (FR)
(72) Inventeur: Samain, Henri, 91570 Bievres (FR); Bauer, Daniel, 93340 Le Raincy (FR)
(74) Mandataire: Andral, Christophe André Louis

(56) Documents cités:
- EP-A- 0 288 012
- EP-A- 0 320 218
- EP-A- 0 323 715
- WO-A-95/28909
- FR-A- 2 697 160

## Description

L'invention concerne une composition cosmétique aqueuse ou hydroalcoolique comprenant une dispersion aqueuse de particules insolubles de polymère non ionique filmogène particulier décrits ci-après, les particules de polymères étant à une concentration supérieure à 15 % en poids et la température de transition vitreuse de l'extrait sec de la composition étant comprise entre 15 et 35°C comme défini dans la revendication 1. L'invention concerne également un procédé de traitement cosmétique à l'aide de ces compositions.

Les compositions capillaires à pulvériser ou à vaporiser sur les cheveux sont essentiellement constituées d'une solution le plus souvent hydroalcoolique et d'un polymère en mélange éventuellement avec divers adjuvants cosmétiques. Cette solution est conditionnée soit dans un flacon pompe, soit dans un récipient aérosol approprié qui est mis sous pression à l'aide d'un gaz propulseur.

Depuis quelques années, un intérêt tout particulier s'est manifesté pour la réalisation de compositions cosmétiques capillaires essentiellement aqueuses. En effet, l'emploi d'alcool tel que l'éthanol ou l'isopropanol, seul ou en mélange avec une faible proportion d'eau, peut présenter certains inconvénients, notamment une augmentation de l'inflammabilité lorsque la composition est sous forme d'une laque aérosol.

De manière encore plus générale, on cherche à réduire l'emploi des composés volatils à la pression atmosphérique, dits COV (Composés Organiques Volatils), qui sont présents dans les compositions cosmétiques. Les COV sont principalement les propulseurs et certains solvants tels que l'éthanol.

Pour diminuer la quantité de COV, on a essayé de remplacer les solvants tels que l'éthanol par de l'eau: Toutefois, si la plupart des polymères filmogènes hydrosolubles peuvent, en solution dans l'eau, conduire à l'obtention de compositions de fixation des cheveux, ces dernières présentent des inconvénients majeurs. Ainsi, les compositions essentiellement aqueuses de ces polymères ne permettent pas d'obtenir de hauts degrés de fixation. Il a certes été proposé d'utiliser ces polymères hydrosolubles à des concentrations élevées, mais l'augmentation de concentration provoque un tel accroissement de la viscosité des compositions que l'on ne peut que très difficilement obtenir une pulvérisation satisfaisante. Même si une pulvérisation correcte est obtenue, ces compositions aqueuses présentent un temps de séchage particulièrement long par rapport aux compositions alcooliques.

Le document EP-A-0 323 715 décrit des produits de coiffage contenant des polymères adhésifs dont la température est, de préférence, comprise entre 30 et 60°C. Dans la description de ce document, il est indiqué, en toutes généralités, que le polymère adhésif est présent dans la composition cosmétique à une concentration comprise entre 0,5 et 25 % en poids.

Le document EP-A-0 320 218 décrit des compositions capillaires comprenant un polymère adhésif dont la température de transition vitreuse est, de manière très générale, comprise entre 30 et 60 °C. Dans la description de ce document, il est indiqué que le polymère adhésif est présent dans les compositions cosmétiques à une concentration comprise entre 0,1 et 20 % en poids environ.

Le document FR-A-2 697 160 décrit des compositions cosmétiques comprenant un polymère filmogène anionique à fonctions acides carboxyliques. Tous les exemples mettent en jeu des polymères anioniques.

Le document WO-A-95/28 909 est un document opposable à la nouveauté de la présente invention au titre de l'article 54(3) CBE. Il décrit des compositions cosmétiques comprenant une dispersion de particules de polymères insolubles. Dans la description, il est indiqué que la concentration en polymères insolubles dans la composition est, de préférence, comprise entre 1 et 20 % en poids. Les exemples de ce document, qui divulguent spécifiquement des compositions à haute concentration en polymère insoluble anionique, ont été considérés au moment du dépôt de la demande de brevet.

Il a également été proposé d'utiliser des dispersions aqueuses de particules insolubles de polymères anioniques à la place des polymères solubles utilisés dans les compositions aqueuses, hydroalcooliques ou alcooliques.
Cependant, jusqu'à présent, les résultats obtenus ne sont pas encore satisfaisants. En effet, le degré dé fixation n'est pas encore suffisant, le temps de séchage est long et les propriétés cosmétiques sont un peu justes.

On recherche donc encore des compositions aqueuses susceptibles de conduire à des propriétés analogues à celles des compositions à forte teneur en alcool à savoir une bonne diffusion lors de l'application, un bon pouvoir de fixation, une résistance à l'humidité, une bonne élimination au shampooing et au brossage, une bonne vitesse de séchage.

La demanderesse a maintenant découvert qu'une composition cosmétique comprenant, dans un milieu cosmétiquement acceptable aqueux ou hydroalcoolique, une dispersion aqueuse de particules de polymères non ioniques insolubles, les particules de polymères étant présentes à une concentration supérieure à 15 % en poids par rapport à l'ensemble de la composition et la température de transition vitreuse de l'extrait sec de la composition étant comprise entre 15 et 35°C, permettait de remédier aux inconvénients décrits ci-dessus.

En particulier, les compositions selon l'invention permettent d'obtenir un bon pouvoir plixant, supérieur à celui obtenu avec une dispersion aqueuse d'un polymère anionique.

La présente invention a donc pour objet une composition cosmétique comprenant, dans un milieu cosmétiquement acceptable aqueux ou hydroalcoolique, une dispersion aqueuse de particules insolubles de polymère non ionique, les particules de polymères étant présentes à une concentration supérieure à 15 % en poids par rapport au poids total de la composition et la température de transition vitreuse de l'extrait sec de la composition étant comprise entre 15 et 35°C,
ladite dispersion aqueuse résultant de la polymérisation ou de la copolymérisation de monomères choisis parmi le styrène, le butadiène, l'éthylène, le propylène, le vinyl toluène, le vinyl propionate, l'alcool vinylique, l'acrylonitrile, le chloroprène, l'acétate de vinyle, les uréthannes, l'isoprène, l'isobutène et les esters ou les amides des acides acrylique ou méthacrylique, maléïque, crotonique ou itaconique, l'éther vinylique, la la vinylpyrrolidone, le vinylimidazole ou le polymère non ionique de la dispersion aqueuse étant choisi parmi les polyesters, les polyamides, les polyuréthannes et les polyéthers ; et
- la composition est conditionnée sous forme d'un spray aérosol, la concentration en propulseur étant comprise entre 10 et 50 % par rapport au poids total de la composition pressurisée ;
   sous réserve que le polymère non ionique soit différent d'un copolymère choisi dans le groupe constitué par :
   (i) - les copolymères acrylate de butyle / méthacrylate de méthyle
   (ii) - les copolymères acrylate de butyle / méthacrylate de méthyle / styrène.

Les compositions selon l'invention présentent, outre les avantages précités, une bonne résistance à l'humidité, une bonne élimination au shampooing et au brossage et une bonne vitesse de séchage.

Mais d'autre caractéristiques, aspects ou avantages de l'invention apparaîtront encore plus complètement à la lecture de la description détaillée qui va suivre et des exemples concrets mais nullement limitatifs destinés à l'illustrer.

Les dispersions aqueuses de particules insolubles de polymère non ionique utilisables selon l'invention sont généralement obtenues par polymérisation ou copolymérisation en suspension ou en émulsion de monomères selon les procédés bien connus de l'état de la technique ( de telles dispersions sont aussi connues sous le nom de "latex").

Les polymères non ioniques de la dispersion aqueuse choisis parmi les polyesters, les polyamides, les polyuréthannes et les polyéthers peuvent provenir de la condensation de monomères ioniques ou non ioniques.

Les dispersions aqueuses particulièrement préférées dans le cadre de l'invention sont les dispersions aqueuses de copolymères styrène/acrylate de butyle tels que par exemple le produit vendu sous la dénomination commerciale URAMUL SC 70 par la société D.S.M. Resins.

La concentration en poids des particules de polymère dans les compositions selon l'invention est de préférence comprise entre 15 et 50% en poids par rapport au poids total de la composition et de préférence entre 15 et 35%.

Le pH des compositions selon l'invention est généralement compris entre 2 et 9, et en particulier entre 3 et 8. Il peut être ajusté à la valeur désirée au moyen d'agents alcalinisants ou acidifiants habituellement utilisés en cosmétique pour ce type d'application.

Lorsque la composition selon l'invention est pressurisée sous forme d'aérosol, l'aérosol comprend la composition décrite ci-dessus, appelée jus, et au moins un agent propulseur qui peut être choisi parmi les hydrocarbures volatils tels que le n-butane, le propane, l'isobutane, le pentane, les hydrocarbures chlorés et/ou fluorés et leurs mélanges. On peut également utiliser en tant qu'agent propulseur le gaz carbonique, le protoxyde d'azote, le diméthyléther, l'azote, l'air comprimé et leurs mélanges.

Les compositions sont pressurisées dans un récipient aérosol afin d'assurer une application de la composition sous forme vaporisée ou sous forme de mousse. De telles formes de conditionnement sont indiquées, par exemple, lorsqu'on souhaite obtenir un spray, une laque ou une mousse pour la fixation des cheveux.

Dans les compositions conditionnées sous forme d'un spray aérosol, la concentration en propulseur(s) est généralement comprise entre 10 et 50% en poids par rapport au poids total de la composition pressurisée et de préférence entre 15 et 35% en poids.

Selon un mode préféré de réalisation de l'invention, la concentration en particules de polymère est d'au moins 10 % en poids par rapport au poids de la composition pressurisée (jus + propulseur), et encore plus préférentiellement comprise entre 10 et 35% en poids.

Les agents propulseurs sont généralement présents dans les mousses aérosol dans des proportions inférieures à 25% en poids par rapport au poids total de la composition et de préférence dans des proportions comprises entre 1 % et 10 %.

Le milieu aqueux ou hydroalcoolique cosmétiquement acceptable, servant de support aux compositions selon l'invention, est de préférence constitué par de l'eau ou une solution hydroalcoolique constitué d'eau et d'un ou plusieurs monoalcools tels que par exemple les alcools inférieurs tels que éthanol, isopropanol ou butanol, d'un ou plusieurs polyalcools, d'un ou plusieurs éthers de glycol qui peuvent être utilisés seuls ou en mélange. Encore plus préférentiellement, ledit support est essentiellement constitué d'eau.

Les compositions selon l'invention (à l'état pressurisée ou non) peuvent encore contenir des agents tensioactifs, des agents conservateurs, des séquestrants, des adoucissants, des parfums, des colorants, des agents modificateurs de viscosité, des des agents modificateurs de mousse, des agents anti-mousse, des agents nacrants, des agents hydratants, des agents antipelliculaires, des agents antiséborrhéiques, des filtres solaires, des protéines, des vitamines, des plastifiants, des hydroxyacides, des électrolytes et des parfums.

Les compositions selon l'invention peuvent également contenir des agents conditionneurs. Ceux-ci peuvent alors être choisis parmi les huiles et les cires naturelles ou synthétiques, les alcools gras, les esters d'alcools polyhydriques, les glycérides, les gommes et résines de silicone ou les mélanges de ces différents composés.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Les compositions utilisées selon l'invention sont par exemple des compositions capillaires rincées ou non rincées.

L'invention a encore pour objet un procédé de traitement cosmétique des matières kératiniques, telles que les cheveux, caractérisé en ce qu'il consiste à appliquer sur matières kératiniques une composition cosmétique telle que définie précédemment, puis à effectuer éventuellement un rinçage à l'eau, après un éventuel temps de pose.

Dans ce qui suit, MA signifie matière active.

### EXEMPLE

On a préparé une composition A selon l'invention et on l'a comparé à une composition B non conforme à l'invention. Ces deux compositions diffèrent l'une de l'autre par la nature du polymère utilisé. Les deux compositions ont été pressurisées en aérosol .

Un panel de 5 testeurs expérimentés a évalué le pouvoir plixant des cheveux après séchage à température ambiante. Le pouvoir plixant est l'aptitude de la composition à maintenir les cheveux dans la forme désirée.

La notation allait de 0 (mauvais) à 5 (excellent).

Les résultats sont rassemblés dans le tableau ci-dessous :

| En gMA | **A (Invention)** | **B (Comparatif)** |
|---|---|---|
| **URAMUL SC 70 (1)** | 18 | - |
| **AMERHOLD DR 25 (2)** | - | 18 |
| **Eau qsp** | 100 | 100 |
| **Pouvoir plixant de la composition pressurisée** | 4,5 | 3 |

| | | |
|---|---|---|
| (1) : URAMUL SC 70 de D.S.M. Resins : Copolymère non ionique styrène/acrylate de butyle (Tg~30°C) en dispersion aqueuse à 50% de matière active | | |
| (2) AMERHOLD DR 25 de AMERCHOL : copolymère anionique acrylate d'éthyle / méthacrylate de méthyle / acide méthacrylique / acide acrylique ayant une température de transition vitreuse d'environ 30°C en dispersion aqueuse à 25% en poids de MA. | | |

Le schéma de pressurisation était le suivant :
- diméthyléther (propulseur) 35 g
- Composition ci-dessus (jus) 65 g

La composition A selon l'invention présente un pouvoir plixant nettement supérieur à celui de la composition B non conforme à l'invention.

## Revendications

1. Composition cosmétique, **caractérisée par le fait qu'**elle comprend, dans un milieu cosmétiquement acceptable aqueux ou hydroalcoolique, une dispersion aqueuse de particules insolubles de polymère non ionique, les particules de polymères étant présentes à une concentration supérieure à 15 % en poids par rapport au poids total de la composition et la température de transition vitreuse de l'extrait sec de la composition étant comprise entre 15 et 35°C,
- ladite dispersion aqueuse résultant de la polymérisation ou de la copolymérisation de monomères choisis parmi le styrène, le butadiène, l'éthylène, le propylène, le vinyl toluène, le vinyl propionate, l'alcool vinylique, l'acrylonitrile, le chloroprène, l'acétate de vinyle, les uréthannes, l'isoprène, l'isobutène et les esters ou les amides des acides acrylique ou méthacrylique, maléïque, crotonique ou itaconique, l'éther vinylique, la vinylpyrrolidone, le vinylimidazole ou le polymère non ionique de la dispersion aqueuse étant choisi parmi les polyesters, les polyamides, les polyuréthannes et les polyéthers ; et
- la composition est conditionnée sous forme d'un spray aérosol, la concentration en propulseur étant comprise entre 10 et 50 % par rapport au poids total de la composition pressurisée ;
sous réserve que le polymère non ionique soit différent d'un copolymère choisi dans le groupe constitué par :
(i) - les copolymères acrylate de butyle / méthacrylate de méthyle
(ii) - les copolymères acrylate de butyle / méthacrylate de méthyle / styrène.

2. Composition selon la revendication 1, **caractérisée par le fait que** ledit polymère non ionique est un copolymère acrylate de butyle / styrène.

3. Composition selon l'une quelconque des revendications précédentes, **caractérisée par le fait que** la concentration en poids des particules de polymère est comprise entre 15 et 50% par rapport au poids total de la composition.

4. Composition selon l'une quelconque des revendications précédentes, **caractérisée par le fait que** la concentration en poids des particules de polymère est comprise entre 15 et 35% par rapport au poids total de la composition.

5. Composition selon l'une quelconque des revendications précédentes, **caractérisée par le fait que** l'agent propulseur est choisi parmi les hydrocarbures volatils tels que le n-butane, le propane, l'isobutane, le pentane, les hydrocarbures chlorés et/ou fluorés, le gaz carbonique, le protoxyde d'azote, le diméthyléther, l'azote, l'air comprimé et leurs mélanges.

6. Composition selon l'une quelconque des revendications précédentes, **caractérisée par le fait que** la concentration en poids des particules de polymère est supérieure à 10% en poids par rapport au poids total de la composition pressurisée.

7. Composition selon la revendication précédente, **caractérisée par le fait que** la concentration en poids des particules de polymère est comprise entre 10 et 35% par rapport au poids total de la composition pressurisée.

8. Composition selon l'une quelconque des revendications précédentes, **caractérisée par** le fait l'agent propulseur est présent dans des concentrations comprises entre 15 et 35% en poids par rapport au poids total de la composition pressurisée.

9. Procédé de traitement cosmétique des matières kératiniques, telles que les cheveux, **caractérisé en ce qu'**il consiste à appliquer sur matières kératiniques une composition cosmétique telle que définie dans l'une quelconque des revendications 1 à 8, puis à effectuer éventuellement un rinçage à l'eau, après un éventuel temps de pose.

## Patentansprüche

1. Kosmetische Zusammensetzung, **dadurch gekennzeichnet, dass** sie in einem kosmetisch akzeptablen, wässrigen oder wässrigalkoholischen Medium eine wässrige Dispersion von unlöslichen Partikeln eines nichtionischen Polymers enthält, wobei die Polymerpartikel in einer Konzentration über 15 Gew.-%, bezogen auf das Gesamtgewicht der Zusammensetzung, vorliegen und die Glasübergangstemperatur des Trockenextraktes der Zusammensetzung im Bereich von 15 bis 35 °C liegt,
- wobei die wässrige Dispersion bei der Polymerisation oder Copolymerisation von Monomeren gebildet wird, die unter Styrol, Butadien, Ethylen, Propylen, Vinyltoluol, Vinylpropionat, Vinylalkohol, Acrylnitril, Chloropren, Vinylacetat, Urethanen, Isopren, Isobuten, Estern oder Amiden von Acrylsäure, Methacrylsäure, Maleinsäure, Crotonsäure oder Itaconsäure, Vinylether, Vinylpyrrolidon und Vinylimidazol ausgewählt sind, oder das nichtionische Polymer der wässrigen Dispersion unter den Polyestern, Polyamiden, Polyurethanen und Polyethern ausgewählt ist; und
- die Zusammensetzung als Aerosolspray konfektioniert ist, wobei die Konzentration des Treibmittels im Bereich von 10 bis 50 %, bezogen auf das Gesamtgewicht der unter Druck stehenden Zusammensetzung, liegt;
mit der Maßgabe, dass das nichtionische Polymer von den Copolymeren verschieden ist, die ausgewählt sind unter:
(i) den Copolymeren Butylacrylat/Methylmethacrylat; und
(ii) den Copolymeren Butylacrylat/Methylmethacrylat/ Styrol.

2. Zusammensetzung nach Anspruch 1, **dadurch gekennzeichnet, dass** das nichtionische Polymer ein Butylacrylat/Styrol-Copolymer ist.

3. Zusammensetzung nach einem der vorhergehenden Ansprüche, **dadurch gekennzeichnet, dass** die Konzentration der Polymerpartikel im Bereich von 15 bis 50 Gew.-%, bezogen auf das Gesamtgewicht der Zusammensetzung, liegt.

4. Zusammensetzung nach einem der vorhergehenden Ansprüche, **dadurch gekennzeichnet, dass** die Konzentration der Polymerpartikel im Bereich von 15 bis 35 Gew.-%, bezogen auf das Gesamtgewicht der Zusammensetzung, liegt.

5. Zusammensetzung nach einem der vorhergehenden Ansprüche, **dadurch gekennzeichnet, dass** das Treibmittel unter den flüchtigen Kohlenwasserstoffen, wie n-Butan, Propan, Isobutan, Pentan, chlorierten und/oder fluorierten Kohlenwasserstoffen, Kohlendioxid, Distickstoffoxid, Dimethylether, Stickstoff, Druckluft und deren Gemischen ausgewählt ist.

6. Zusammensetzung nach einem der vorhergehenden Ansprüche, **dadurch gekennzeichnet, dass** die Konzentration der Polymerpartikel über 10 Gew.-%, bezogen auf das Gesamtgewicht der unter Druck stehenden Zusammensetzung, liegt.

7. Zusammensetzung einem dem vorhergehenden Anspruch, **dadurch gekennzeichnet, dass** die Konzentration der Polymerpartikel im Bereich von 10 bis 35 Gew.-%, bezogen auf das Gesamtgewicht der unter Druck stehenden Zusammensetzung, liegt.

8. Zusammensetzung nach einem der vorhergehenden Ansprüche, **dadurch gekennzeichnet, dass** das Treibmittel in Konzentrationen von 15 bis 35 Gew.-%, bezogen auf das Gesamtgewicht der unter Druck stehenden Zusammensetzung, vorliegt.

9. Verfahren zur kosmetischen Behandlung von Keratinsubstanzen, wie dem Haar, **dadurch gekennzeichnet, dass** es darin besteht, auf die Keratinsubstanzen eine kosmetische Zusammensetzung nach einem der Ansprüche 1 bis 8 aufzubringen und dann gegebenenfalls nach einer Einwirkzeit gegebenenfalls mit Wasser zu spülen.

## Claims

1. Cosmetic composition, **characterized in that** it comprises, in a cosmetically acceptable aqueous or aqueous/alcoholic medium, an aqueous dispersion of insoluble non-ionic polymer particles, the polymer particles being present at a concentration greater than 15% by weight with respect to the total weight of the composition and the glass transition temperature of the solids content of the composition being between 15 and 35°C,
- the said aqueous dispersion resulting from the polymerization or from the copolymerization of monomers chosen from styrene, butadiene, ethylene, propylene, vinyltoluene, vinyl propionate, vinyl alcohol, acrylonitrile, chloroprene, vinyl acetate, urethanes, isoprene, isobutene and the esters or the amides of acrylic or methacrylic, maleic, crotonic or itaconic acids, vinyl ether, vinylpyrrolidone or vinylimidazole, or the non-ionic polymer of the aqueous dispersion being chosen from polyesters, polyamides, polyurethanes and polyethers; and
- the composition being packaged in the form of an aerosol spray, the concentration of propellant being between 10 and 50% with respect to the total weight of the pressurized composition;
with the proviso that the nonionic polymer is other than a copolymer chosen from the group consisting of:
(i) - butyl acrylate/methyl methacrylate copolymers
(ii) - butyl acrylate/methyl methacrylate/styrene copolymers.

2. Composition according to Claim 1, **characterized in that** the said nonionic polymer is a butyl acrylate/styrene copolymer.

3. Composition according to either one of the preceding claims, **characterized in that** the concentration by weight of the polymer particles is between 15 and 50% with respect to the total weight of the composition.

4. Composition according to any one of the preceding claims, **characterized in that** the concentration by weight of the polymer particles is between 15 and 35% with respect to the total weight of the composition.

5. Composition according to any one of the preceding claims, **characterized in that** the propellant agent is chosen from volatile hydrocarbons, such as n-butane, propane, isobutane or pentane, chlorinated and/or fluorinated hydrocarbons, carbon dioxide gas, nitrous oxide, dimethyl ether, nitrogen, compressed air and mixtures thereof.

6. Composition according to any one of the preceding claims, **characterized in that** the concentration by weight of the polymer particles is greater than 10% by weight with respect to the total weight of the pressurized composition.

7. Composition according to the preceding claim, **characterized in that** the concentration by weight of the polymer particles is between 10 and 35% with respect to the total weight of the pressurized composition.

8. Composition according to any one of the preceding claims, **characterized in that** the propellant agent is present in concentrations of between 15 and 35% by weight with respect to the total weight of the pressurized composition.

9. Process for the cosmetic treatment of keratinous substances, such as hair, **characterized in that** it consists in applying, to keratinous substances, a cosmetic composition as defined in any one of Claims 1 to 8 and in then optionally rinsing with water, after an optional setting time.
